# EUROPEAN PATENT APPLICATION

(11) **EP 4 461 151 A1**
(43) Date of publication of application: **13.11.2024**
(21) Application number: 22933323.2
(22) Date of filing: 23.03.2022
(51) Int. Cl.: A24F 40/57

(54) **INFORMATION PROCESSING DEVICE, INFORMATION PROCESSING METHOD, AND PROGRAM**

(71) Applicant: Japan Tobacco Inc., Tokyo 105-6927 (JP)
(72) Inventor: YAMADA, Manabu, Tokyo 130-8603 (JP)
(74) Representative: Hoffmann Eitle
(86) International application number: PCT/JP2022/013535
(87) International publication number: WO 2023/181179

(57) **Abstract**

[Problem] To provide a mechanism for customization by which a user can easily realize a desired flavor.

[Solution] Provided is an information processing device comprising a control unit that, in accordance with a user operation, changes control information which is used by an aerosol generating device that heats an aerosol source included in a base material to generate an aerosol and which stipulates a time series transition in a parameter relating to a temperature at which the aerosol source is heated. If a user operation for changing the parameter at a first time stipulated by the control information has been implemented, the control unit sets a second time earlier than the first time and changes the control information such that the change in the parameter begins at the second time and arrives at the changed parameter at the first time.

## Description

### Technical Field

The present invention relates to an information processing device, an information processing method, and a program.

### Background Art

Inhaler devices that generate a substance to be inhaled by users, such as electronic cigarettes and nebulizers, are widely used. An inhaler device generates an aerosol with a flavor component, for example, using a substrate including an aerosol source for generating an aerosol and a flavor source for imparting a flavor component to the generated aerosol. A user can taste a flavor by inhaling the aerosol with the flavor component generated by the inhaler device. Inhalation of an aerosol by the user will be referred to as a "puff" or a "puff action" hereinafter.

A preference for a flavor (hereinafter also referred to as a smoke taste) tasted during a puff differs between users. Heating temperature of an aerosol source, which directly affects the smoke taste, therefore, is preferably customizable by a user. The following Patent Literature 1 discloses a technique for enabling a user to customize heating temperature of an aerosol source.

### Citation List

### Patent Literature

Patent Literature 1: WO 2019/104227 A1

### Summary of Invention

### Technical Problem

With the technique disclosed in the above Patent Literature 1, however, there are cases where it is difficult for a user to perform customization as desired. This is because there is a complex relationship between heating temperature of an aerosol source and a smoke taste.

The present invention, therefore, has been conceived in view of the above problem, and aims to provide a mechanism capable of easily achieving smoke tastes desired by a user.

### Solution to Problem

In order to solve the above problem, an aspect of the present invention provides an information processing device including a controller that changes control information, which is used by an aerosol generation device that generates an aerosol by heating an aerosol source included in a substrate and which defines temporal changes in a parameter relating to heating temperature of the aerosol source, in accordance with a user operation. When a user operation for changing the parameter at a first time defined by the control information is performed, the controller sets a second time, which is earlier than the first time, and changes the control information such that the parameter starts to change at the second time and reaches the parameter after the change at the first time.

The controller may set the second time on a basis of the change of the parameter at the first time.

The controller may set the second time to a time with which a rate of change in the parameter from the second time to the first time becomes lower than or equal to a certain threshold.

The controller may increase an interval between the first time and the second time as an amount of change in the parameter at the first time becomes larger and decrease the interval as the amount of change becomes smaller.

The controller may set the interval between the first time and the second time on a basis of a characteristic of the aerosol generation device.

The controller may set an interval between the first time and the second time on a basis of a type of substrate.

When a user operation for changing the parameter at the first time defined by the control information is performed, the controller may set a third time, which is later than the first time, and change the control information such that the change in the parameter continues from the first time to the third time and ends at the third time.

The controller may set the third time on a basis of the change of the parameter at the first time.

The controller may set the third time to a time with which a rate of change in the parameter from the first time to the third time becomes lower than or equal to a certain threshold.

The controller may increase an interval between the first time and the third time as an amount of change in the parameter at the first time becomes larger and decrease the interval as the amount of change becomes smaller.

The controller may set the interval between the first time and the third time on a basis of a characteristic of the aerosol generation device.

The controller may set the interval between the first time and the third time on a basis of a type of substrate.

The first time may be one of a plurality of certain times in a period of time when the aerosol is generated using the control information.

The information processing device may further include an inputter that receives the user operation relating to a change of the parameter each time a certain time comes in the period of time when the aerosol generation device generates the aerosol using the control information. The controller may change the control information on a basis of the user operation received by the inputter.

In addition, in order to solve the above problem, another aspect of the present invention provides an information processing method including changing control information, which is used by an aerosol generation device that generates an aerosol by heating an aerosol source included in a substrate and which defines temporal changes in a parameter relating to heating temperature of the aerosol source, in accordance with a user operation. The changing control information includes setting, when a user operation for changing the parameter at a first time defined by the control information is performed, a second time, which is earlier than the first time, and changing the control information such that the parameter starts to change at the second time and reaches the parameter after the change at the first time.

In addition, in order to solve the above problem, another aspect of the present invention provides a program for causing a computer to function as a controller that changes control information, which is used by an aerosol generation device that generates an aerosol by heating an aerosol source included in a substrate and which defines temporal changes in a parameter relating to heating temperature of the aerosol source, in accordance with a user operation. When a user operation for changing the parameter at a first time defined by the control information is performed, the controller sets a second time, which is earlier than the first time, and changes the control information such that the parameter starts to change at the second time and reaches the parameter after the change at the first time.

### Advantageous Effects of Invention

As described above, according to the present invention, a customization mechanism capable of easily achieving smoke tastes desired by a user is provided.

### Brief Description of Drawings

[Fig. 1] Fig. 1 is a diagram for describing a configuration example of a system according to an embodiment.
[Fig. 2] Fig. 2 is a schematic diagram schematically illustrating a configuration example of an inhaler device according to the present embodiment.
[Fig. 3] Fig. 3 is a block diagram illustrating a configuration example of a terminal device according to the present embodiment.
[Fig. 4] Fig. 4 is a graph schematically illustrating an example of a heating profile.
[Fig. 5] Fig. 5 is a graph schematically illustrating an example of customization of the heating profile.
[Fig. 6] Fig. 6 is a graph schematically illustrating another example of the customization of the heating profile.
[Fig. 7] Fig. 7 is a flowchart illustrating an example of a procedure of a process for customizing a heating profile performed by the terminal device according to the present embodiment.

### Description of Embodiments

A preferred embodiment of the present invention will be described in detail hereinafter with reference to the accompanying drawings. Structural elements having substantially the same functional configuration will be given the same reference numerals herein and in the drawings, and redundant description thereof is omitted.

### <1. Configuration example>

### (1) System configuration example

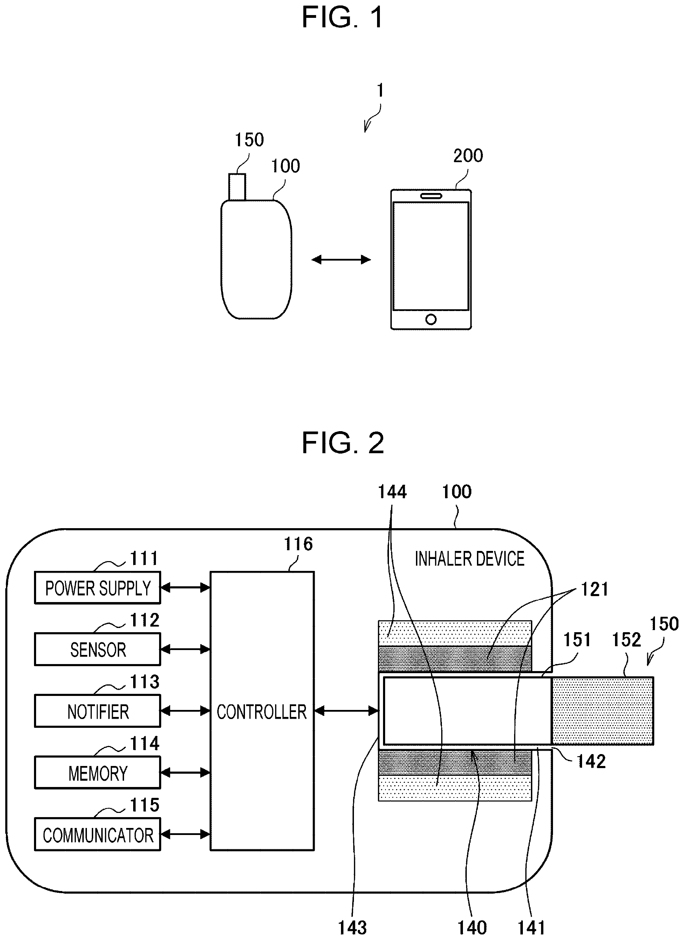

Fig. 1 is a diagram for describing a configuration example of a system 1 according to an embodiment. As illustrated in Fig. 1, the system 1 includes an inhaler device 100 and a terminal device 200.

The inhaler device 100 is a device that generates a substance to be inhaled by a user. It is assumed in the following description that the substance generated by the inhaler device 100 is an aerosol. Alternatively, the substance generated by the inhaler device may be a gas. The inhaler device 100 generates the aerosol using a stick substrate 150. The stick substrate 150 is an example of a substrate including an aerosol source. The inhaler device 100 is an example of an aerosol generation device that generates an aerosol by heating an aerosol source included in a substrate.

The terminal device 200 is an information processing device that performs various types of information processing relating to the inhaler device 100. The user of the inhaler device 100 uses the terminal device 200. The terminal device 200 may be any device such as a smartphone, a tablet terminal, a wearable device, or a personal computer (PC). Alternatively, the terminal device 200 may be a charger for charging the inhaler device 100.

The terminal device 200 is used to change settings of the inhaler device 100. For example, the terminal device 200 receives a user operation for changing the settings of the inhaler device 100 and changes the settings of the inhaler device 100.

### (2) Configuration example of inhaler device

Fig. 2 is a schematic diagram schematically illustrating a configuration example of the inhaler device according to the present embodiment. As illustrated in Fig. 2, the inhaler device 100 includes a power supply 111, a sensor 112, a notifier 113, a memory 114, a communicator 115, a controller 116, a heater 121, a container 140, and a heat insulator 144.

The power supply 111 stores electric power. The power supply 111 supplies electric power to the structural elements of the inhaler device 100 under the control of the controller 116. The power supply 111 may be a rechargeable battery such as a lithium ion secondary battery.

The sensor 112 acquires various items of information regarding the inhaler device 100. In an example, the sensor 112 may be a pressure sensor such as a condenser microphone, a flow sensor, or a temperature sensor, and acquire a value generated in accordance with the user's inhalation. In another example, the sensor 112 may be an input device that receives information input by the user, such as a button or a switch.

The notifier 113 provides information for the user. The notifier 113 may be a light-emitting device that emits light, a display device that displays an image, a sound output device that outputs sound, or a vibration device that vibrates.

The memory 114 stores various items of information for operation of the inhaler device 100. The memory 114 may be a non-volatile storage medium such as flash memory.

The communicator 115 is a communication interface capable of communication in conformity with any wired or wireless communication standard. Such a communication standard may be, for example, Wi-Fi (registered trademark), Bluetooth (registered trademark), near-field communication (NFC), or a standard using a low-power wide-area network (LPWA).

The controller 116 functions as an arithmetic processing unit and a control circuit, and controls the overall operations of the inhaler device 100 in accordance with various programs. The controller 116 is achieved by an electronic circuit such as a central processing unit (CPU) or a microprocessor, for example.

The container 140 has an internal space 141, and holds the stick substrate 150 in a manner partially accommodated in the internal space 141. The container 140 has an opening 142 that allows the internal space 141 to communicate with outside. The container 140 accommodates the stick substrate 150 that is inserted into the internal space 141 through the opening 142. For example, the container 140 may be a tubular body having the opening 142 and a bottom 143 on its ends, and may define the pillar-shaped internal space 141. The container 140 connects with an airflow path that supplies air to the internal space 141. For example, a side surface of the inhaler device 100B has an air inlet hole that is an inlet of air into the airflow path. For example, the bottom 143 has an air outlet hole that is an outlet of the air from the airflow path to the internal space 141.

The stick substrate 150 includes a substrate 151 and an inhalation port 152. The substrate 151 includes an aerosol source. For example, the aerosol source may be a liquid such as polyhydric alcohol or water. Examples of the polyhydric alcohol include glycerine and propylene glycol. The aerosol source may include the flavor component that is either derived from tobacco or not derived from tobacco. For the inhaler device 100 that is a medical inhaler such as a nebulizer, the aerosol source may include a medicine. In this configuration example, the aerosol source is not limited to a liquid, and may be a solid, instead. The stick substrate 150 held by the container 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the airflow path (not illustrated), and the air and an aerosol generated from the substrate 151 reach inside the mouth of the user.

The heater 121 heats the aerosol source to atomize the aerosol source and generate the aerosol. In the example illustrated in Fig. 2, the heater 121 has a film-like shape and surrounds the outer circumference of the container 140. Subsequently, heat produced from the heater 121 heats the substrate 151 of the stick substrate 150 from the outer circumference, generating the aerosol. The heater 121 produces heat when receiving electric power from the power supply 111. In an example, the electric power may be supplied in response to the sensor 112 detecting a start of the user's inhalation and/or an input of predetermined information. Subsequently, the supply of the electric power may be stopped in response to the sensor 112 detecting an end of the user's inhalation and/or an input of predetermined information.

The heat insulator 144 prevents heat from transferring from the heater 121 to the other structural elements. For example, the heat insulator 144 may be a vacuum heat insulator, an aerogel heat insulator, or the like.

The configuration example of the inhaler device 100 has been described above. It is needless to say that the inhaler device 100 is not limited to the above configuration, and may be configured in various ways as exemplified below.

In an example, the heater 121 may have a blade-like shape, and may be disposed so that the heater 121 protrudes from the bottom 143 of the container 140 toward the internal space 141. In this case, the heater 121 having the blade-like shape is inserted into the substrate 151 of the stick substrate 150 and heats the substrate 151 of the stick substrate 150 from its inside. In another example, the heater 121 may be disposed so that the heater 121 covers the bottom 143 of the container 140. In still another example, the heater 121 may be implemented as a combination of two or more selected from a first heater that covers the outer circumference of the container 140, a second heater having the blade-like shape, and a third heater that covers the bottom 143 of the container 140.

In another example, the container 140 may include an opening/closing mechanism that at least partially opens and closes an outer shell defining the internal space 141. Examples of the opening/closing mechanism include a hinge. In addition, the container 140 may sandwich the stick substrate 150 inserted into the internal space 141 by opening and closing the outer shell. In this case, the heater 121 may be provided at the sandwiching position of the container 140 and produce heat while pressing the stick substrate 150.

In addition, means for atomizing the aerosol source is not limited to the heating by the heater 121. For example, the means for atomizing the aerosol source may be induction heating, instead. In this case, the inhaler device 100 includes at least an electromagnetic induction source, such as a coil, that generates a magnetic field instead of the heater 121. The inhaler device 100 may be provided with a susceptor that produces heat through induction heating, or the stick substrate 150 may include the susceptor.

The inhaler device 100 generates the aerosol to be inhaled by the user by operating in conjunction with the stick substrate 150. A combination of the inhaler device 100 and the stick substrate 150, therefore, may be regarded as an aerosol generation system.

### (3) Configuration example of terminal device

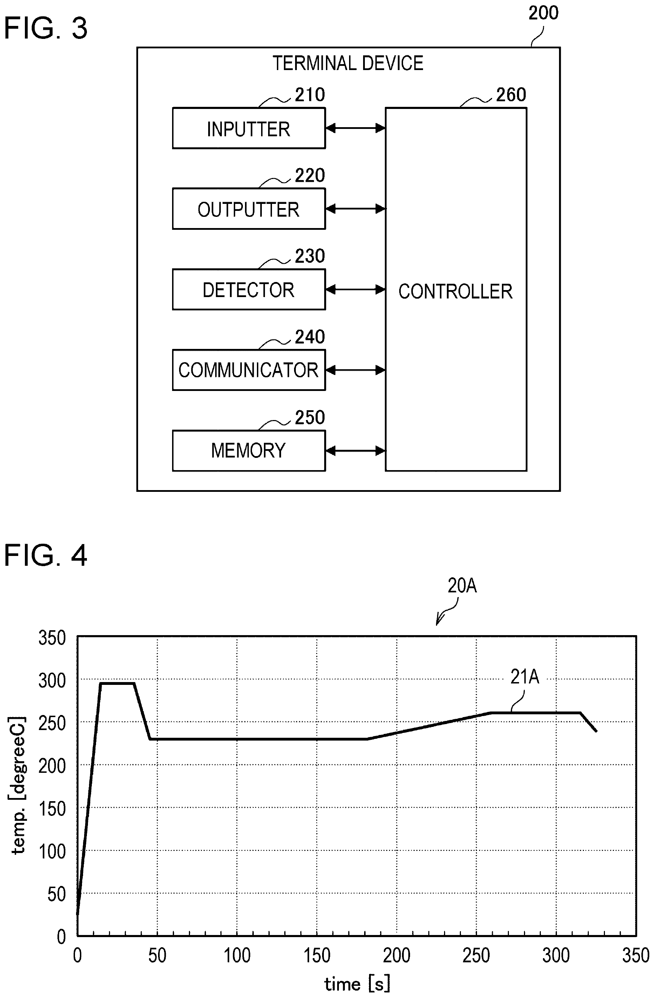

Fig. 3 is a block diagram illustrating a configuration example of the terminal device 200 according to the present embodiment. As illustrated in Fig. 3, the terminal device 200 includes an inputter 210, an outputter 220, a detector 230, a communicator 240, a memory 250, and a controller 260.

The inputter 210 has a function of receiving inputs of various items of information. The inputter 210 may include an input device that receives inputs of information from the user. The input device may be, for example, buttons, a keyboard, a touch panel, a microphone, or the like. The inputter 210 may also include various sensors including an image sensor.

The outputter 220 has a function of outputting information. The outputter 220 may include an output device that outputs information for the user. The output device may be, for example, a display device that displays information, a light-emitting device that emits light, a vibration device that vibrates, a sound output device that outputs sound, or the like. An example of the display device is a display. An example of the light-emitting device is a light-emitting diode (LED). An example of the vibration device is an eccentric motor. An example of the sound output device is a speaker. The outputter 220 provides information for the user by outputting information input from the controller 260.

The detector 230 has a function of detecting information regarding the terminal device 200. The detector 230 may detect positional information regarding the terminal device 200. For example, the detector 230 receives a global navigation satellite system (GNSS) signal from a GNSS satellite (e.g., a global positioning system (GPS) signal from a GPS satellite) and detects positional information regarding a device including latitude and longitude. The detector 230 may detect movement of the terminal device 200. For example, the detector 230 includes a gyro sensor and an acceleration sensor and detects angular velocity and acceleration.

The communicator 240 is a communication interface for communicating information between the terminal device 200 and other devices. The communicator 240 performs communication in conformity with any wired or wireless communication standard. Such a communication standard may be, for example, universal serial bus (USB), Wi-Fi (registered trademark), Bluetooth (registered trademark), near-field communication (NFC), or a standard using a low-power wide-area network (LPWA).

The memory 250 stores various items of information. The memory 250 is achieved, for example, by a nonvolatile storage medium such as a flash memory.

The controller 260 functions as an arithmetic processing unit and a control circuit, and controls the overall operations of the terminal device 200 in accordance with various programs. The controller 260 is achieved by, for example, an electronic circuit such as a central processing unit (CPU) or a microprocessor. The controller 260 may also include a read-only memory (ROM) storing programs to be used, operation parameters, and the like and a random-access memory (RAM) that temporarily stores parameters which change as appropriate and the like. The terminal device 200 performs various types of processing under the control of the controller 260. Examples of the processing controlled by the controller 260 include the processing of information input using the inputter 210, the outputting of information from the outputter 220, the detection of information by the detector 230, the communication of information by the communicator 240, and the storing and the reading of information by the memory 250. The controller 260 also controls other types of processing performed by the terminal device 200 including inputting of information to each structural element, processing based on information output from each structural elements, and the like.

The functions of the controller 260 may be achieved using an application. The application may be preinstalled or downloaded. Alternatively, the functions of the controller 260 may be achieved by progressive web Apps (PWAs).

### <2. Technical features>

### (1) Heating profile

The controller 116 controls the operation of the heater 121 on the basis of a heating profile. The control of the operation of the heater 121 is achieved by controlling the supply of power from the power supply 111 to the heater 121. The heater 121 heats the stick substrate 150 using the power supplied from the power supply 111.

The heating profile is control information for controlling heating temperature of the aerosol source. The heating profile defines a parameter relating to the heating temperature of the aerosol source. An example of the heating temperature of the aerosol source is temperature of the heater 121. An example of the parameter relating to the heating temperature of the aerosol source is a target value of the temperature (hereinafter also referred to as a target temperature) of the heater 121. The temperature of the heater 121 may be controlled in such a way as to change in accordance with time elapsed since a start of heating. In this case, the heating profile includes information that defines temporal changes in the target temperature. In another example, the heating profile can include a parameter that defines a method for supplying power to the heater 121 (hereinafter referred to as a power supply parameter). The power supply parameter includes, for example, a voltage applied to the heater 121, on/off of the supply of power to the heater 121, a feedback control method to be employed, or the like. On/off of the supply of power to the heater 121 may be regarded as on/off of the heater 121.

The controller 116 controls the operation of the heater 121 such that the temperature (hereinafter also referred as an actual temperature) of the heater 121 changes in the same manner as the target temperature defined by the heating profile. The heating profile is typically designed in such a way as to optimize the flavor tasted by the user when the user inhales the aerosol generated from the stick substrate 150. By controlling the operation of the heater 121 on the basis of the heating profile, therefore, the flavor tasted by the user can be optimized.

The control of the temperature of the heater 121 can be achieved through, for example, known feedback control. The feedback control may be, for example, a proportional-integral-differential (PID) controller. The controller 116 can supply the power from the power supply 111 to the heater 121 in a form of a pulse based on pulse width modulation (PWM) or pulse frequency modulation (PFM). In this case, the controller 116 can control the temperature of the heater 121 by adjusting a duty ratio of the power pulse or frequency in the feedback control. Alternatively, the controller 116 may perform simple on/off control in the feedback control. For example, the controller 116 may cause the heater 121 to produce heat until the actual temperature reaches the target temperature, stop producing heat when the actual temperature reaches the target temperature, and resume the heating when the actual temperature falls below the target temperature.

The temperature of the heater 121 can be quantified by, for example, measuring or estimating an electrical resistance of the heater 121 (more specifically, a heating resistor included in the heater 121). This is because the electrical resistance of the heating resistor changes in accordance with the temperature. The electrical resistance of the heating resistor can be estimated by measuring a decrease in voltage of the heating resistor. The decrease in the voltage of the heating resistor can be measured by a voltage sensor that measures a potential difference applied to the heating resistor. In another example, the temperature of the heater 121 can be measured by a temperature sensor, such as a thermistor, provided near the heater 121.

A period of time from a start to an end of a process for generating the aerosol using the stick substrate 150 will be referred to as a heating session hereinafter. In other words, the heating session is a period of time in which the supply of power to the heater 121 is controlled on the basis of the heating profile. The start of the heating session is a time when heating based on the heating profile starts. The end of the heating session is a time when a sufficient amount of aerosol is no longer generated. The heating session includes a preheating period in a first half and a puffable period in a second half. The puffable period is a period when a sufficient amount of aerosol is assumed to be generated. The preheating period is a period from a start of heating until the puffable period starts. Heating performed in the preheating period will also be referred to as preheating.

The notifier 113 may provide, for the user, information indicating a time when the preheating will end. For example, the notifier 113 provides information indicating an end of the preheating before the preheating ends and provides information indicating that the preheating has ended when the preheating has ended. The provision of information for the user can be performed, for example, through lighting of the LED, vibration, or the like. The user can puff immediately after the end of the preheating on the basis of the provision of information.

Similarly, the notifier 113 may provide, for the user, information indicating a time when the puffable period will end. For example, the notifier 113 provides information indicating an end of the puffable period before the puffable period ends and provides information indicating that the puffable period has ended when the puffable period has ended. The provision of information for the user can be performed, for example, through lighting of the LED, vibration, or the like. The user can puff until the puffable period ends on the basis of the provision of information.

An example of the heating profile will be described with reference to Fig. 4. Fig. 4 is a graph schematically illustrating an example of the heating profile. A horizontal axis of a graph 20A represents time (seconds). A vertical axis of the graph 20A represents the target temperature. A line 21A indicates temporal changes in the target temperature. As illustrated in Fig. 4, the target temperature may sharply increase to around 300°C after a start of heating, decrease to around 230°C, and then increase to around 260°C again. When the heating based on the heating profile is performed, the temperature of the heater 121 sharply increases to around 300°C after the start of the heating, decreases to around 230°C, and then increases to around 260°C again.

### (2) Customization of heating profile

The controller 260 changes a heating profile used by the inhaler device 100 in accordance with a user operation. More specifically, when a user operation for changing a target temperature at a first time defined in the heating profile is performed, the controller 260 changes the target temperature at the first time in accordance with the user operation. If a user operation for increasing a target temperature 60 seconds after a start of heating by 10°C is performed, for example, the controller 260 changes the heating profile such that the target temperature 60 seconds after the start of the heating increases by 10°C. With this configuration, the user can customize the heating profile as desired.

There are cases where it is difficult to achieve a smoke taste desired by the user even if a heating profile is changed as requested by the user. This is because there is a time lag between when the temperature of the heater 121 increases or decreases and when a smoke taste actually changes.

The terminal device 200 according to the present embodiment, therefore, performs customization that takes into consideration such a time lag in addition to a request from the user. That is, when a user operation for changing a target temperature at a first time defined in a heating profile is performed, the controller 260 sets a second time, which is earlier than the first time. The controller 260 then changes the heating profile such that a target temperature starts to change at the second time and then, at the first time, reaches a target temperature after the change. It is assumed, for example, that a user operation for increasing a target temperature 60 seconds after a start of heating by 10°C has been performed. In this case, the controller 260 may increase the target temperature 60 seconds after the start of the heating by 10°C and also increase target temperatures in a period from 50 seconds to 60 seconds after the start of the heating. At this time, the controller 260 desirably increases the amount of increase in the target temperature gradually from 50 seconds to 60 seconds after the start of heating. As a result, the target temperature gradually increases from 50 seconds after the start of the heating, and the amount of increase in the target temperature reaches 10°C 60 seconds after the start of heating. With this configuration, the temperature of the heater 121 increases prior to a time specified by the user. As a result, a smoke taste can be actually changed at a time when the user desires to change the smoke taste. This is because the smoke taste reflects an effect of a change in the temperature of the heater 121 with a delay due to the above-described time lag.

The controller 260 may set the second time on the basis of a change of the target temperature at the first time. For example, the controller 260 sets the second time in accordance with at least whether the target temperature at the first time has been increased or decreased or the amount of change in the target temperature. With this configuration, appropriate measures against the time lag based on a mode of the customization can be taken.

More specifically, the controller 260 may set the second time to a time with which a rate of change in the target temperature from the second time to the first time becomes lower than or equal to a certain threshold. That is, the controller 260 may set a time sufficiently earlier than the first time as the second time so that the rate of change in the target temperature becomes lower than or equal to the certain threshold. With this configuration, the rate of change in the temperature of the heater 121 can be reduced. As a result, an event where an unsubtle smoke taste is delivered to the user due to a sudden change in the temperature of the heater 121 can be prevented.

In particular, the controller 260 may increase an interval between the first time and the second time as the amount of change in the target temperature at the first time becomes larger, and decrease the interval as the amount of change becomes smaller. When the amount of change in the target temperature at the first time is 10°C, for example, the controller 260 may set a time 10 seconds before the first time as the second time. When the amount of change in the target temperature at the first time is 20°C, on the other hand, the controller 260 may set a time 20 seconds before the first time as the second time. With this configuration, changes in the temperature of the heater 121 can be made gradual.

In addition, the controller 260 may set the interval between the first time and the second time on the basis of characteristics of the inhaler device 100. For example, the controller 260 may set a time earlier than the first time by a length corresponding to characteristics of the heater 121 included in the inhaler device 100 as the second time. With this configuration, the rate of change in the temperature of the heater 121 can be made low enough for the heater 121 to be able to operate stably. As a result, an event where an unsubtle smoke taste is delivered to the user due to instable changes in the temperature of the heater 121 can be prevented.

In addition, the controller 260 may set the interval between the first time and the second time on the basis of a type of stick substrate 150. For example, the controller 260 may set a time earlier than the first time by a length corresponding to whether the stick substrate 150 includes menthol as the second time. With this configuration, the rate of change in the temperature of the heater 121 can be made low enough to generate an aerosol to which an appropriate flavor is imparted. As a result, an event where an unsubtle flavor is delivered to the user can be prevented.

Here, the change of the target temperature at the first time can also affect smoke tastes after the first time. If the change in the target temperature ends at the first time and the temperature of the heater 121 immediately returns to an original target temperature, an unsubtle flavor might be delivered to the user due to a sudden change in the temperature of the heater 121.

The terminal device 200 according to the present embodiment, therefore, performs customization for making a change in the temperature of the heater 121 gradual in addition to a request from the user. That is, when a user operation for changing a target temperature at a first time defined in a heating profile is performed, the controller 260 sets a third time, which is later than the first time. The controller 260 then changes the heating profile such that the change in the target temperature continues from the first time to the third time and ends at the third time. It is assumed, for example, that a user operation for increasing a target temperature 60 seconds after a start of heating by 10°C has been performed. In this case, the controller 260 may increase the target temperature 60 seconds after the start of the heating by 10°C and also increase target temperatures in a period from 60 seconds to 70 seconds after the start of the heating. At this time, the controller 260 desirably decreases the amount of increase in the target temperature from 60 seconds to 70 seconds after the start of the heating. As a result, the target temperature that has increased by 10°C gradually decreases to an original target temperature from 60 seconds to 70 seconds after the start of the heating. With this configuration, a change in the temperature of the heater 121 after a time specified through a user operation can be made gradual. As a result, an event where an unsubtle flavor is delivered to the user due to a sudden change in the temperature of the heater 121 can be prevented.

The controller 260 may set the third time on the basis of the change of the target temperature at the first time. For example, the controller 260 sets the third time in accordance with at least whether the target temperature at the first time has been increased or decreased or the amount of change in the target temperature. With this configuration, return time, which is time taken for the temperature of the heater 121 to return to the original target temperature, can be secured in accordance with the mode of the customization.

More specifically, the controller 260 may set the third time to a time with which a rate of change in the target temperature from the first time to the third time becomes lower than or equal to a certain threshold. That is, the controller 260 may set a time sufficiently later than the first time as the third time so that the rate of change in the target temperature becomes lower than or equal to the certain threshold. With this configuration, the change in the temperature of the heater 121 can be made gradual. As a result, an event where an unsubtle flavor is delivered to the user due to a sudden change in the temperature of the heater 121 can be prevented.

In particular, the controller 260 may increase an interval between the first time and the third time as the amount of change in the target temperature at the first time becomes larger and decrease the interval as the amount of change becomes smaller. When the amount of change in the target temperature at the first time is 10°C, for example, the controller 260 may set a time 10 seconds after the first time as the third time. When the amount of change in the target temperature at the first time is 20°C, on the other hand, the controller 260 may set a time 20 seconds after the first time as the third time. With this configuration, a sufficiently long return time corresponding to the amount of change in the target temperature at the first time can be secured. As a result, the change in the temperature of the heater 121 can be made gradual.

In addition, the controller 260 may set the interval between the first time and the third time on the basis of the characteristics of the inhaler device 100. For example, the controller 260 may set a time later than the third time by a length corresponding to the characteristics of the heater 121 included in the inhaler device 100 as the third time. With this configuration, the rate of change in the temperature of the heater 121 can be made low enough for the heater 121 to be able to operate stably. As a result, an event where an unsubtle flavor is delivered to the user due to instable changes in the temperature of the heater 121 can be prevented.

In addition, the controller 260 may set the interval between the first time and the third time on the basis of a type of stick substrate 150. For example, the controller 260 may set a time later than the first time by a length corresponding to whether the stick substrate 150 includes menthol as the third time. With this configuration, the rate of change in the temperature of the heater 121 can be made low enough to generate an aerosol to which an appropriate flavor is imparted. As a result, an event where an unsubtle flavor is delivered to the user can be prevented.

The first time may be one of a plurality of certain times in a heating session. In an example, the certain times may be times when the user puffs (hereinafter referred to as puff times). Each time a puff time comes, that is, each time the user puffs, for example, the user performs a user operation for changing a target temperature in accordance with a smoke taste. As a result, the user can change smoke tastes at puff times as desired.

The second time, too, may be one of the plurality of times in the heating session. The same holds for the third time.

Each time a puff time comes in a heating session, the inputter 210 may receive a user operation relating to a change of a target temperature. The controller 260 may then change a heating profile on the basis of the user operation received by the inputter 210. In an example, each time a puff time comes in a heating session, the outputter 220 may display a screen for requesting the user to input an evaluation of a smoke taste. The user may then input the evaluation of the smoke taste to the inputter 210 in accordance with the request. Furthermore, with respect to a puff time at which a negative evaluation has been input as an evaluation of a smoke taste, the outputter 220 may display a screen for requesting the user to input a change of a target temperature. The user may then input the change of the targe temperature in accordance with the request. The inputting of a change of a target temperature may be performed at each puff time (i.e., immediately after a negative evaluation is input as an evaluation of a smoke taste) or performed later after the heating session ends. With this configuration, the user can intuitively change a heating profile while puffing.

Puff times may be defined in advance. In this case, each time a puff time comes, the outputter 220 may display a screen for urging the user to puff. Alternatively, puff times need not be defined in advance. In this case, each time the inhaler device 100 detects the user's puff, the outputter 220 may display a screen for requesting the user to input an evaluation of a smoke taste and/or a change of a target temperature.

### (3) Specific examples

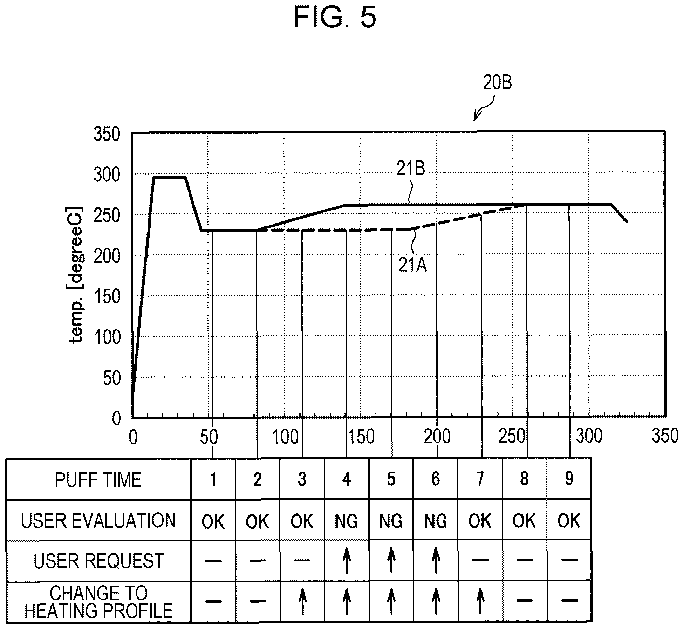

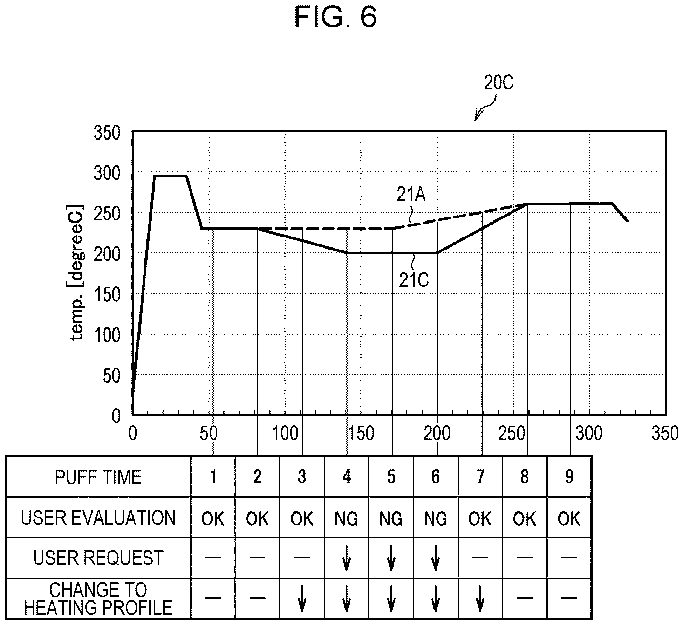

Specific examples of customization of the heating profile illustrated in Fig. 4 will be described with reference to Figs. 5 and 6.

### - First specific example

Fig. 5 is a graph schematically illustrating an example of the customization of the heating profile. A horizontal axis of a graph 20B represents time (seconds). A vertical axis of the graph 20B represents the target temperature. The line 21A indicates the temporal changes in the target temperature before the customization. A line 21B indicates temporal changes in the target temperature after the customization.

In the example illustrated in Fig. 5, a total of nine puff times are defined in advance. Each time a predefined puff time comes while the inhaler device 100 is performing the heating based on the heating profile indicated by the line 21A, the terminal device 200 urges the user to puff, and receives an evaluation of a smoke taste. In the figure, "OK" indicates a positive evaluation, and "NG" indicates a negative evaluation. Inputting of a negative evaluation corresponds to requesting a change of a target temperature. In the example illustrated in Fig. 5, negative evaluations have been input at fourth to sixth puff times. The terminal device 200, therefore, receives an input of changes of target temperatures at the fourth to sixth puff times. In the example illustrated in Fig. 5, a request to increase the target temperatures at the fourth to sixth times has been input.

The terminal device 200, therefore, increases the target temperatures at the fourth to sixth times as indicated by the line 21B. Furthermore, the terminal device 200 also increases a target temperature at a third puff time as indicated by the line 21B. This is because the fourth puff time corresponds to the first time and the third puff time corresponds to the second time. The terminal device 200 also increases a target temperature at a seventh puff time as indicated by the line 21B. This is because the sixth puff time corresponds to the first time and the seventh puff time corresponds to the third time. As a result, the target temperatures at the fourth to sixth puff times can be increased from those before the customization, and changes in temperature before and after the fourth to sixth puff times can be made gradual.

### - Second specific example

Fig. 6 is a graph schematically illustrating another example of the customization of the heating profile. A horizontal axis of a graph 20C represents time (seconds), and a vertical axis of the graph 20C represents the target temperature. The line 21A indicates the temporal changes in the targe temperature before the customization. A line 21C indicates temporal changes in the target temperature after the customization.

In the example illustrated in Fig. 6, a total of nine puff times are defined in advance. Each time a predefined puff time comes while the inhaler device 100 is performing the heating based on the heating profile indicated by the line 21A, the terminal device 200 urges the user to puff, and receives an evaluation of a smoke taste. In the example illustrated in Fig. 6, negative evaluations have been input at the fourth to sixth puff times. The terminal device 200, therefore, receives an input of changes of the target temperatures at the fourth to sixth puff times. In the example illustrated in Fig. 6, a request to decrease the target temperatures at the fourth to sixth puff times has been input.

The terminal device 200, therefore, decreases the target temperatures at the fourth to sixth puff times as indicated by the line 21C. The terminal device 200 also decreases the target temperature at the third puff time as indicated by the line 21C. This is because the fourth puff time corresponds to the first time, and the third puff time corresponds to the second time. The terminal device 200 also decreases the target temperature at the seventh puff time as indicated by the line 21C. This is because the sixth puff time corresponds to the first time, and the seventh puff time corresponds to the third time. As a result, the target temperatures at the fourth to sixth puff times can be decreased compared to those before the customization, and changes in temperature before and after the fourth to sixth puff times can be made gradual.

### (4) Procedure of process

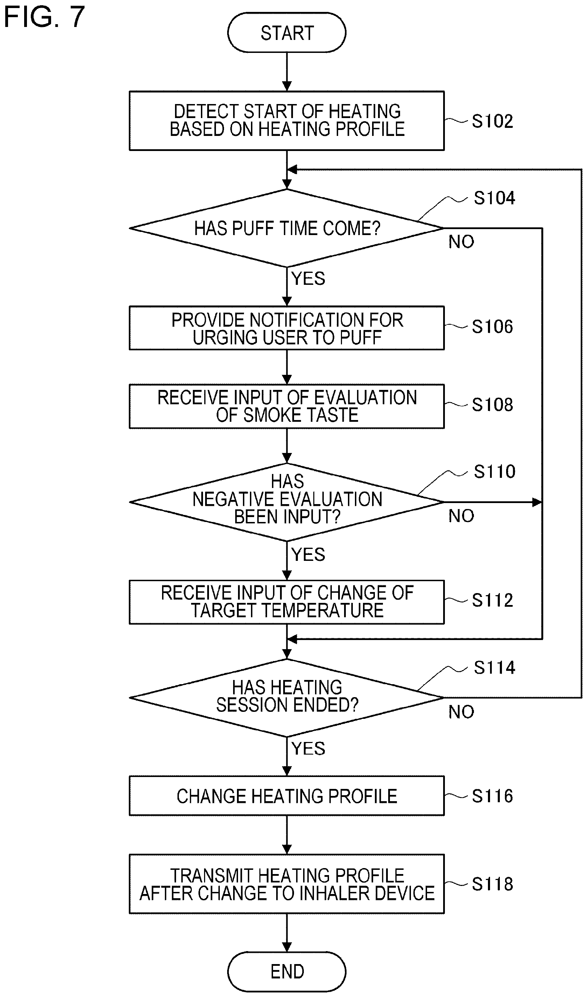

A procedure of a process for customizing a heating profile according to the present embodiment will be described hereinafter with reference to Fig. 7. Fig. 7 is a flowchart illustrating an example of a procedure of a process for customizing a heating profile performed by the terminal device 200 according to the present embodiment.

As illustrated in Fig. 7, first, the controller 260 detects a start of heating based on a heating profile (step S102). When heating based on a heating profile has started, for example, the inhaler device 100 may transmit, to the terminal device 200, information indicating that the heating has started. Upon receiving the information, the controller 260 can detect the start of the heating based on the heating profile.

Next, the controller 260 determines whether a puff time has come (step S104). In an example, the controller 260 determines whether a predefined puff time has come on the basis of time elapsed since the start of the heating.

If the controller 260 determines that a puff time has not come (NO in step S104), the process proceeds to step S 114.

If determining that a puff time has come (YES in step S 104), the controller 260 controls the outputter 220 such that the outputter 220 provides a notification for urging the user to puff (step S106). For example, the outputter 220 may display a screen for urging the user to puff.

Next, the inputter 210 receives an input of an evaluation of a smoke taste (step S108). For example, the outputter 220 displays an input screen for receiving an input indicating whether the smoke taste is good or bad. The inputter 210 then receives an input to the input screen.

Next, the controller 260 determines whether a negative evaluation has been input (step 5110).

If the controller 260 determines that a negative evaluation has been input (YES in step S110), the inputter 210 receives an input of a change of a target temperature (step S112). For example, the outputter 220 displays an input screen for receiving an input of a change of a target temperature. The inputter 210 then receives an input to the input screen. The process then proceeds to step S 114.

If the controller 260 determines that a positive evaluation has been input (NO in step S110), the process proceeds to step S114.

In step S114, the controller 260 determines whether the heating session has ended (step S114). For example, the inhaler device 100 may transmit information indicating that the heating session has ended to the terminal device 200 when the heating based on the heating profile has ended. Upon receiving the information, the controller 260 can detect the end of the heating session.

If the controller 260 determines that the heating session has not ended (NO in step S114), the process returns to step S104.

If determining that the heating session has ended (YES in step S114), the controller 260 changes the heating profile on the basis of the change of the target temperature input during the heating session (step S116). At this time, the controller 260 changes not only the target temperature at the puff time (i.e., the first time) when the change of the target temperature has been input but also target temperatures at puff times before and/or after the foregoing puff time (i.e., the second time and/or the third time).

Thereafter, the controller 260 controls the communicator 240 such that the communicator 240 transmits the heating profile after the change to the inhaler device 100 (step S118). As a result, the inhaler device 100 can perform heating based on the heating profile after the change.

The above-described step S112 may be performed between step S114 and step S116, instead. That is, the inhaler device 100 may receive an input of changes of target temperatures after the heating session ends (YES in step S1 14), instead of receiving an input of a change of a target temperature each time the controller 260 determines in step S110 that a negative evaluation has been input. With this configuration, the user can collectively input changes of target temperatures after a heating session ends.

### <3. Supplementary information>

Although a preferred embodiment of the present invention has been described in detail with reference to the accompanying drawings, the present invention is not limited to this example. It is clear that those who have ordinary knowledge in a technical field to which the present invention pertains can conceive various examples of alterations or modifications within the scope of the technical idea described in the claims, and it is understood that these also naturally belong to the technical scope of the present invention.

Although an example where the heater 121 is implemented as a heating resistor and produces heat using electrical resistance has been described in the above embodiment, the present invention is not limited to this example. For example, the heater 121 may include an electromagnetic induction source that generates a magnetic field, such as a coil, and a susceptor that produces heat through induction heating, and the susceptor may heat the stick substrate 150, instead. In this case, the controller 116 applies an alternating current to the electromagnetic induction source to generate an alternating magnetic field, and heats the susceptor by applying the alternating magnetic field to the susceptor. In this case, the heating temperature of the aerosol source, which is controlled on the basis of a heating profile, is temperature of the susceptor. The temperature of the susceptor can be estimated on the basis of an electrical resistance of the electromagnetic induction source.

Although an example where the parameter relating to the heating temperature of the aerosol source defined in a heating profile is the target temperature of the heater 121 has been described in the above embodiment, the present invention is not limited to this example. The parameter relating to the heating temperature of the aerosol source may be the electrical resistance of the heater 121, instead of the temperature of the heater 121 described in the above embodiment. When the inhaler device 100 includes an electromagnetic induction source instead of the heater 121, the parameter relating to the heating temperature of the aerosol source defined in a heating profile may be target values of the temperature of the susceptor or the electrical resistance of the electromagnetic induction source.

Although an example where the inhaler device 100 heats the stick substrate 150 to generate an aerosol has been described in the above embodiment, the present invention is not limited to this example. The inhaler device 100 may be an aerosol generation device of a so-called liquid atomization type, which generates an aerosol by heating and atomizing an aerosol source as a liquid, instead. The present invention can be applied to aerosol generation devices of the liquid atomization type.

Each device described herein may be achieved as an independent device, or part or the entirety thereof may be achieved as separate devices. For example, the controller 260 of the terminal device 200 may be included in an apparatus, such as a server, connected to the terminal device 200 over a network or the like. That is, the customization of a heating profile may be performed by a cloud server on the basis of a user operation input to the terminal device 200.

It is to be noted that the process by each device described herein may be achieved by software, hardware, or a combination of software and hardware. A program constituting software is stored in advance, for example, in a storage medium (more specifically, a non-transitory computer-readable storage medium) provided inside or outside each device. When executed by a computer that controls each device described herein, for example, each program is loaded into a RAM and executed by a processing circuit such as CPU. The storage medium is, for example, a magnetic disk, an optical disc, a magneto-optical disk, a flash memory, or the like. In addition, the computer program may be distributed over a network, instead, without using a storage medium. In addition, the computer may be an integrated circuit for a specific application such as an ASIC, a general-purpose processor that executes a function by reading a software program, a computer on a server used for cloud computing, or the like. In addition, the process by each device described herein may be performed by a plurality of computers in a distributed manner.

In addition, the process described herein with reference to the flowchart and the sequence diagram need not necessarily be performed in the illustrated order. Some processing steps may be performed in parallel with each other, instead. Additional processing steps may also be employed, or some processing steps may be omitted.

The following configurations also belong to the technical scope of the present invention.
(1) An information processing device including:
   a controller that changes control information, which is used by an aerosol generation device that generates an aerosol by heating an aerosol source included in a substrate and which defines temporal changes in a parameter relating to heating temperature of the aerosol source, in accordance with a user operation,
   in which, when a user operation for changing the parameter at a first time defined by the control information is performed, the controller sets a second time, which is earlier than the first time, and changes the control information such that the parameter starts to change at the second time and reaches the parameter after the change at the first time.
(2) The information processing device according to (1),
   in which the controller sets the second time on a basis of the change of the parameter at the first time.
(3) The information processing device according to (2),
   in which the controller sets the second time to a time with which a rate of change in the parameter from the second time to the first time becomes lower than or equal to a certain threshold.
(4) The information processing device according to (2) or (3),
   in which the controller increases an interval between the first time and the second time as an amount of change in the parameter at the first time becomes larger and decreases the interval as the amount of change becomes smaller.
(5) The information processing device according to any of (1) to (4),
   in which the controller sets the interval between the first time and the second time on a basis of a characteristic of the aerosol generation device.
(6) The information processing device according to any of (1) to (5),
   in which the controller sets an interval between the first time and the second time on a basis of a type of substrate.
(7) The information processing device according to any of (1) to (6),
   in which, when a user operation for changing the parameter at the first time defined by the control information is performed, the controller sets a third time, which is later than the first time, and changes the control information such that the change in the parameter continues from the first time to the third time and ends at the third time.
(8) The information processing device according to (7),
   in which the controller sets the third time on a basis of the change of the parameter at the first time.
(9) The information processing device according to (8),
   in which the controller sets the third time to a time with which a rate of change in the parameter from the first time to the third time becomes lower than or equal to a certain threshold.
(10) The information processing device according to (8) or (9),
   in which the controller increases an interval between the first time and the third time as an amount of change in the parameter at the first time becomes larger and decreases the interval as the amount of change becomes smaller.
(11) The information processing device according to any of (8) to (10),
   in which the controller sets the interval between the first time and the third time on a basis of a characteristic of the aerosol generation device.
(12) The information processing device according to any of (8) to (11),
   in which the controller sets the interval between the first time and the third time on a basis of a type of substrate.
(13) The information processing device according to any of (1) to (12),
   in which the first time is one of a plurality of certain times in a period of time when the aerosol is generated using the control information.
(14) The information processing device according to (13), further including:
   an inputter that receives the user operation relating to a change of the parameter each time a certain time comes in the period of time when the aerosol generation device generates the aerosol using the control information,
   in which the controller changes the control information on a basis of the user operation received by the inputter.
(15) An information processing method including:
   changing control information, which is used by an aerosol generation device that generates an aerosol by heating an aerosol source included in a substrate and which defines temporal changes in a parameter relating to heating temperature of the aerosol source, in accordance with a user operation,
   in which the changing control information includes setting, when a user operation for changing the parameter at a first time defined by the control information is performed, a second time, which is earlier than the first time, and changing the control information such that the parameter starts to change at the second time and reaches the parameter after the change at the first time.
(16) A program for causing a computer to function as:
   a controller that changes control information, which is used by an aerosol generation device that generates an aerosol by heating an aerosol source included in a substrate and which defines temporal changes in a parameter relating to heating temperature of the aerosol source, in accordance with a user operation,
   in which, when a user operation for changing the parameter at a first time defined by the control information is performed, the controller sets a second time, which is earlier than the first time, and changes the control information such that the parameter starts to change at the second time and reaches the parameter after the change at the first time.

### Reference Signs List

- 1: system
- 100: inhaler device
- 111: power supply
- 112: sensor
- 113: notifier
- 114: memory
- 115: communicator
- 116: controller
- 121: heater
- 140: container
- 141: internal space
- 142: opening
- 143: bottom
- 144: heat insulator
- 150: stick substrate
- 151: substrate
- 152: inhalation port
- 200: terminal device
- 210: inputter
- 220: outputter
- 230: detector
- 240: communicator
- 250: memory
- 260: controller

## Claims

1. An information processing device comprising:
a controller that changes control information, which is used by an aerosol generation device that generates an aerosol by heating an aerosol source included in a substrate and which defines temporal changes in a parameter relating to heating temperature of the aerosol source, in accordance with a user operation,
wherein, when a user operation for changing the parameter at a first time defined by the control information is performed, the controller sets a second time, which is earlier than the first time, and changes the control information such that the parameter starts to change at the second time and reaches the parameter after the change at the first time.

2. The information processing device according to claim 1,
wherein the controller sets the second time on a basis of the change of the parameter at the first time.

3. The information processing device according to claim 2,
wherein the controller sets the second time to a time with which a rate of change in the parameter from the second time to the first time becomes lower than or equal to a certain threshold.

4. The information processing device according to claim 2 or 3,
wherein the controller increases an interval between the first time and the second time as an amount of change in the parameter at the first time becomes larger and decreases the interval as the amount of change becomes smaller.

5. The information processing device according to any of claims 1 to 4,
wherein the controller sets the interval between the first time and the second time on a basis of a characteristic of the aerosol generation device.

6. The information processing device according to any of claims 1 to 5,
wherein the controller sets an interval between the first time and the second time on a basis of a type of substrate.

7. The information processing device according to any of claims 1 to 6,
wherein, when a user operation for changing the parameter at the first time defined by the control information is performed, the controller sets a third time, which is later than the first time, and changes the control information such that the change in the parameter continues from the first time to the third time and ends at the third time.

8. The information processing device according to claim 7,
wherein the controller sets the third time on a basis of the change of the parameter at the first time.

9. The information processing device according to claim 8,
wherein the controller sets the third time to a time with which a rate of change in the parameter from the first time to the third time becomes lower than or equal to a certain threshold.

10. The information processing device according to claim 8 or 9,
wherein the controller increases an interval between the first time and the third time as an amount of change in the parameter at the first time becomes larger and decreases the interval as the amount of change becomes smaller.

11. The information processing device according to any of claims 8 to 10,
wherein the controller sets the interval between the first time and the third time on a basis of a characteristic of the aerosol generation device.

12. The information processing device according to any of claims 8 to 11,
wherein the controller sets the interval between the first time and the third time on a basis of a type of substrate.

13. The information processing device according to any of claims 1 to 12,
wherein the first time is one of a plurality of certain times in a period of time when the aerosol is generated using the control information.

14. The information processing device according to claim 13, further comprising:
an inputter that receives the user operation relating to a change of the parameter each time a certain time comes in the period of time when the aerosol generation device generates the aerosol using the control information,
wherein the controller changes the control information on a basis of the user operation received by the inputter.

15. An information processing method comprising:
changing control information, which is used by an aerosol generation device that generates an aerosol by heating an aerosol source included in a substrate and which defines temporal changes in a parameter relating to heating temperature of the aerosol source, in accordance with a user operation,
wherein the changing control information includes setting, when a user operation for changing the parameter at a first time defined by the control information is performed, a second time, which is earlier than the first time, and changing the control information such that the parameter starts to change at the second time and reaches the parameter after the change at the first time.

16. A program for causing a computer to function as:
a controller that changes control information, which is used by an aerosol generation device that generates an aerosol by heating an aerosol source included in a substrate and which defines temporal changes in a parameter relating to heating temperature of the aerosol source, in accordance with a user operation,
wherein, when a user operation for changing the parameter at a first time defined by the control information is performed, the controller sets a second time, which is earlier than the first time, and changes the control information such that the parameter starts to change at the second time and reaches the parameter after the change at the first time.
